# EUROPÄISCHE PATENTSCHRIFT

(11) **EP 3 744 277 B1**
(45) Veröffentlichungstag und Bekanntmachung des Hinweises auf die Patenterteilung: **11.09.2024**
(21) Anmeldenummer: 19176733.4
(22) Anmeldetag: 27.05.2019
(51) Int. Cl.: A61B 18/12, A61B 5/24, A61B 5/00, A61N 1/36

(54) **VERFAHREN UND VORRICHTUNG ZUR QUANTIFIZIERUNG NEUROMUSKULÄRER REIZUNGEN DURCH HF-STRÖME**
METHOD AND DEVICE FOR QUANTIFYING NEUROMUSCULAR INFLAMMATION DUE TO HF FLOWS
PROCÉDÉ ET DISPOSITIF DE QUANTIFICATION D'IRRITATIONS NEUROMUSCULAIRES PAR COURANT HF

(43) Veröffentlichungstag der Anmeldung: 02.12.2020
(73) Patentinhaber: Erbe Elektromedizin GmbH, 72072 Tübingen (DE)
(72) Erfinder: Biber, Ulrich, 72770 Reutlingen (DE); Jurjut, Ovidiu, 72070 Tuebingen (DE); Enderle, Markus D., 72070 Tuebingen (DE)
(74) Vertreter: Rüger Abel Patentanwälte PartGmbB

(56) Entgegenhaltungen:
- WO-A1-2004/016315
- 10: "PRO Lesson A03 - COMPOUND ACTION POTENTIAL: NERVE CONDUCTION Using the frog sciatic nerve", 23 October 2015 (2015-10-23), XP055639823, Retrieved from the Internet <URL:https://www.biopac.com/wp-content/uploads/a03.pdf> [retrieved on 20191107]
- LEI CHEN ET AL: "Experiment on building a real-time temperature field distribution model of the prostate using special data encryption multi-pole radiofrequency ablation and a visualization phantom", CHINESE SCIENCE BULLETIN, vol. 56, no. 35, 3 December 2011 (2011-12-03), CN, pages 3845 - 3853, XP055639928, ISSN: 1001-6538, DOI: 10.1007/s11434-011-4777-4
- STAVROS G DEMOS ET AL: "Real time assessment of RF cardiac tissue ablation with optical spectroscopy", OPTICS EXPRESS, 12 September 2008 (2008-09-12), pages 15286 - 15295, XP055639995, Retrieved from the Internet <URL:https://www.osapublishing.org/DirectPDFAccess/B705C6F2-98E8-C30F-A17C6B6796778EF8_172027/oe-16-19-15286.pdf?da=1&id=172027&seq=0&mobile=no> [retrieved on 20191107]

## Beschreibung

Die Erfindung betrifft ein Verfahren zur Quantifizierung neuromuskulärer Reizungen außerhalb eines lebenden Organismus sowie eine Einrichtung zur Durchführung des Verfahrens gemäß den beigefügten Ansprüchen.

Es ist bekannt, dass von elektrochirurgischen Instrumenten neuromuskuläre Stimulationen ausgehen können. Zwar werden elektrochirurgische Instrumente typischerweise mit einer Hochfrequenzspannung beaufschlagt, deren Frequenz oberhalb der Stimulationsschwelle für Nerven liegt, jedoch lassen sich damit neuromuskuläre Reizungen nicht sicher ausschließen. Unter anderem können solche neuromuskulären Reizungen bei der transurethralen Elektroresektion der Harnblase sowie bei der transurethralen Elektroresektion der Blase oder der Prostata in der Urologie und bei der hysteroskopischen transzervikalen Resektion in der Gynäkologie auftreten. Zum Beispiel können bei einer Resektion eines Tumors der Harnwege mögliche intraoperative Komplikationen, u.a. Blutungen bis hin zu Blasenperforationen auftreten. Typischerweise ist dies die Konsequenz einer Stimulation des Obturatornervs und der damit einhergehenden plötzlichen und heftigen Beinadduktorenkontraktion. Eine solche Blasenwandperforation stellt ein in jedem Fall zu vermeidendes Ereignis dar. Bekannte Möglichkeiten zur Vermeidung bestehen in der pharmakologische Hemmung der Erregbarkeit des Obturatornervs, einer tieferen Relaxation der Skelettmuskulatur des Patienten oder dem Betrieb des Instruments mit sehr niedrigem Energieeintrag. Jede dieser Maßnahmen ist mit spezifischen Nachteilen verknüpft, sodass sie nicht als allgemeine Abhilfe zur Vermeidung neuromuskulärer Stimulationen empfohlen werden kann. Es gilt deshalb, Einflussfaktoren neuromuskulärer Stimulationen zu erforschen, um diese bei der Konzeption von Instrumenten, Generatoren und Operationsvorschriften beachten zu können.

Aus der Veröffentlichung "Pro Lesson A03-Compound Action Potential: Nerve Conduction using the frog sciatic nerv", 23. Oktober 2015, XP055639823 ist die Ableitung von Signalen von einem Froschnerven bekannt, der im Abstand zu der Ableitstelle mit einem elektrischen Impuls erregt worden ist. Dazu wird der präparierte Nerv auf eine Anzahl parallel zueinander angeordneter elektrisch leitfähiger Pins aufgelegt, von denen einige zur Einleitung von Signalen und andere zur Ausleitung von Signalen aus dem Nerven dienen können.
Weiter ist aus Lei Chen at al: Experiment on Building a Real-Time Temperature Field Distribution Model of the Prostate Using Special Data Encription, Multi-Pole, Radiofrequency Ablation and a Visualization Phantom", Chinese Science Bulletin, Bd. 56, Nr.353 Dezember 2011, Seiten 3845 bis 3853, XP055639928, ein Verfahren zur Untersuchung der Wirkung einer HF-Elektrode auf ein Prostata Phantom bekannt. Damit sollte die Wärmeausbreitung in Prostatagewebe untersucht werden, das zuvor Hunden entnommen worden ist.
Aus Stavros G. Demos et al: Real Time Assessment of RF Cardiac Tissue Ablation with Optical Spectroscopy", Optics Express, 12. September 2008, Seiten 15286 bis 15295, XP055639995 ist die Untersuchung von zur Laboruntersuchung von HF-Ablationsprozessen an Herzgewebe bekannt. Aus einer SchlAchterei bezogenes Herzgewebe wird dazu in einem mit Blut oder Natriumchloridlösung gefüllten Gefäß angeordnet und mit einen HF-Katheter abladiert. Das entstehende Licht wird spektrographisch untersucht.
Weiter ist aus der WO2004/016315 A1 ein Neurostimulator bekannt, der einen akustischen Transducer und ein Elektrodenpaar aufweist. Während der akustische Transducer zur Erzeugung einer Druckwelle dient, können die Elektroden einen Stimulationsstrom in den Nerv einleiten. Mit dieser Anordnung lassen sich Anregungseffekte auf Nerven untersuchen.

Es ist Aufgabe der Erfindung, ein Konzept zur reproduzierbaren Bestimmung neuromuskulärer Stimulationen bei gewählten Instrumenten, Operationsmoden, Generatoreinstellungen und Operationstechniken zu schaffen.

Diese Aufgabe wird mit dem Verfahren nach Anspruch 1 und mit der Testeinrichtung nach Anspruch 11 gelöst.

Das erfindungsgemäße Verfahren nutzt eine Instrumententestkammer zu reproduzierbaren Erprobung eines Instruments und zur Aufzeichnung von elektrischen Signalen, die in der Instrumententestkammer bei aktiviertem Instrument aufgenommen werden können. Das Verfahren nutzt weiter eine Nerventestkammer, in der mit Hilfe der Instrumententestkammer gewonnene, gegebenenfalls hinsichtlich ihrer Amplitude normierte und/oder hinsichtlich ihres Frequenzgehalts gefilterte oder anderweitig verarbeitete Signale zur Stimulation eines Nervmodells genutzt und die sich daraus ergebenden Summenaktionspotentiale gemessen werden. Das Nervmodell kann ein mathematisches, durch ein Computerprogramm realisiertes Modell, ein durch Hardware realisiertes digiltales oder analoges Modell oder ein präparierter Nerv sein. Die Erfindung gestattet die Quantifizierung neuromuskulärer Reaktionen bei Anwendung elektrochirurgischer Systeme. Unter einem solchen elektrochirurgischem System wird die Gesamtheit, bestehend aus dem für die Operation vorgesehenen Instrument, die gewählte Spannungsform der von dem Generator an das Instrument abgegebene HF-Spannung und -Leistung sowie die Art des elektrochirurgischen Einsatzes, beispielsweise die Schnittführung verstanden. Durch das erfindungsgemäße Verfahren zur Quantifizierung neuromuskulärer Reizungen außerhalb eines lebenden Organismus kann bereits vor der Verwendung am Tier oder Menschen das Risiko neuromuskulärer Stimulationen durch ein elektrochirurgisches System beziffert werden. Es wird somit eine Plattform geschaffen, die es grundsätzlich ermöglicht, das Risiko neuromuskulärer Stimulationen beim neuen elektrochirurgischen System auf ein klinisch akzeptables Niveau einzustellen, ohne dazu Tierversuche durchführen zu müssen. Dies gestattet die Beschleunigung der Entwicklung elektrochirurgischer Systeme bei zugleich erhöhter Sicherheit.

Die Erfindung beruht auf der Trennung der Untersuchung des elektrochirurgischen Systems von der Untersuchung der Auswirkung auf einen präparierten Nerven oder ein Nervmodell. Zur Untersuchung des elektrochirurgischen Systems gehört die Instrumententestkammer, in der das zu untersuchende Instrument platziert und mit einer HF-Spannung vorgegebener Kurvenform (Wellenform) beaufschlagt und auf vorgegebene Weise positioniert wird. Das Positionieren des Instruments kann das Anordnen desselben an einem vorgegebenen Ort der Instrumentenmesskammer beinhalten. Das Positionieren kann ebenso gut das Bewegen des aktivierten, d.h., mit HF-Spannung beaufschlagten Instruments entlang eines vorgegebenen Pfads beinhalten.

In der Instrumententestkammer wird mindestens ein elektrisches Signal aufgenommen, aus dem ein Anregungssignal ermittelt wird. Das Anregungssignal wird an das Nervmodell übergeben, das so mit dem Anregungssignal stimuliert wird und dementsprechend Summenaktionspotentiale abgeben kann. Letztere werden erfasst und zur Bewertung der neuromuskulären Stimulation genutzt. Dazu kann es beispielsweise in einer Datenbank zusammen mit den sonstigen Versuchsbedingungen (Instrumententyp, HF-Spannungsform, Anregungsspannung oder Leistung, Operationsweg) abgespeichert werden.

Das Nervmodell kann wie erwähnt ein technisches Modell (elektrische analoge oder digitale Schaltung oder Computerprogramm) sein. Alternativ kann das Nervmodell durch eine Nerven-Testkammer gebildet sein, die mindestens ein Stimulationselektrodenpaar und mindestens ein Ableitelektrodenpaar aufweist, wobei in der Nerventestkammer ein präparierter funktionsfähiger Nerv so platziert ist, dass er sowohl Stimulationselektrodenpaar als auch die Ableitelektrodeenpaar berührt, um an diesem Summenaktionspotentiale zu messen. Das in der Instrumententestkammer aufgenommene elektrische Signal kann zunächst gespeichert oder auch direkt zur Ermittlung des Anregungssignals verarbeitet werden. Bedarfsweise kann zusätzlich oder alternativ das ermittelte Anregungssignal gespeichert werden. Die Verarbeitung des elektrischen Signals zur Ermittlung des Anregungssignals kann eine Filterung, insbesondere eine Tiefpassfilterung, und/oder eine zeitliche Skalierung und/oder eine Amplitudenskalierung umfassen. Zur Amplitudenskalierung kann das Anregungssignal normiert werden, indem es mit einer Referenzreizspannung abgeglichen wird. Dazu kann ein Standardsignal, beispielsweise 7V Stimulationsamplitude Rechteckpuls mit einer Dauer von 100 ps, als Stimulationssignal an das Nervmodel abgegeben und daraus das Summenaktionspotential mit maximaler Antwortamplitude bestimmt werden. In einem nächsten Schritt kann dasjenige Stimulationspotential bestimmt werden, das ausreicht, um ein gerade noch messbares Summenaktionspotential zu erhalten. Dadurch wird die Responsivität des Nervs anhand von erfahrungsgemä-ßen Grenzwerten kontrolliert. In einem weiteren Schritt kann das im Versuch durch Einsatz eines realen Instruments in der Messkammer gewonnene Anregungssignal verstärkt oder abgeschwächt werden bis es mit dem durch den Testimpuls gewonnenen Summenaktionspotential oder einem festgelegten Bruchteil desselben entspricht. Das Anregungssignal wird dabei vorzugsweise auf eine Amplitude eingestellt, die ein Summenaktionspotential ergibt, das kleiner ist als das maximale Summenaktionspotential jedoch größer als das an einem Nervmodell zu messende minimale Summenaktionspotential. Das erfindungsgemäße Verfahren kann auch beinhalten, dass das von dem Nervmodell gelieferte Summenaktionspotential weiter verarbeitet wird bevor es zur Bewertung eines Operationssystems herangezogen wird. Dazu kann in der Nerven-Testkammer ein funktionsunfähiger, z.B. einem Tier entnommener Nerv angeordnet werden, der mit dem gleichen Anregungssignal beaufschlagt wird, wie der funktionsfähige Nerv. Das von dem funktionsunfähigen Nerven gelieferte Signal kann von den Summenaktionspotentialen des funktionsfähigen Nerven subtrahiert werden, um eine nicht von der Funktion des Nerven herrührende Signalübertragung von dem Anregungssignal aus den Summenaktionspotentialen herauszurechnen.

Der funktionsunfähige Nerv kann ein präparierter Nerv mit einer Quetschstelle sein, die zwischen dem Stimulationselektrodenpaar und dem Ableitelektrodenpaar platziert ist.

Die ebenfalls zur Erfindung gehörige Einrichtung zur Quantifizierung neuromuskulärer Reizungen außerhalb eines lebenden Organismus ist zur Durchführung des erfindungsgemäßen Verfahrens geeignet und eingerichtet. Dazu weist diese Einrichtung insbesondere eine Instrumententestkammer auf, die eine erste Abteilung und eine zweite Abteilung aufweist, die voneinander durch eine Wand getrennt und jeweils wenigstens teilweise mit gleichen oder unterschiedlichen Flüssigkeiten gefüllt sind. Die Flüssigkeit in zumindest einem der Räume, vorzugsweise dem zweiten, ist vorzugsweise eine Elektrolyt, insbesondere Kochsalzlösung. Während in der ersten Abteilung das Instrument angeordnet und gegebenenfalls bewegt wird sind in der zweiten Abteilung Ableitelektroden positioniert. Die Wand zur Trennung der Räume kann zum Beispiel durch ein aus einem Organismus explantiertes Gewebe beispielsweise eine Blasenwand gebildet sein. Es ist jedoch auch möglich, die Wand aus einem anderen Naturstoff oder Kunststoff auszubilden, um reproduzierbare Ergebnisse zu liefern.

Zu der Einrichtung gehört außerdem eine Verarbeitungseinheit, der die von den Ableitelektroden abgegebenen Signale zugeleitet sind. Diese Signale können von den Ableitelektroden direkt an die Verarbeitungseinheit gegeben oder zuvor zwischengespeichert werden. In letzterem Fall ist zwischen den Ableitelektroden und der Verarbeitungseinheit ein Speichergerät angeordnet.

Die Verarbeitungseinheit kann zur Skalierung, Normierung und/oder Filterung der von den Ableitelektroden gelieferten elektrischen Signale dienen, um daraus Anregungssignale für das Nervenmodell zu bilden.

Mit dem vorgestellten Verfahren und der vorgestellten Einrichtung ist es möglich, Operationssysteme unter reproduzierbaren Verhältnissen zu testen und die Auswirkung von Modifikationen hinsichtlich Instrumentengestaltung und/oder HF-Spannungsform und/oder Energieeintrag und/oder Bewegung des Instruments zu erforschen. Dabei ist es möglich, verschiedene Operationssysteme gegenüber zustellen und beispielsweise hinsichtlich der zu verwendenden Instrumente und deren Elektrodenformen, der zu verwendenden HF-Spannung und der Kurvenform derselben sowie hinsichtlich der Schnittführung zu vergleichen.

In der Zeichnung ist eine Einrichtung zur Quantifizierung neuromuskulärer Reizungen außerhalb eines lebenden Organismus veranschaulicht. Es zeigen:
Figur 1 ein Instrument-Testkammer mit zugehörigen Komponenten und
Figur 2 ein Nervmodell mit zugehörigen Komponenten, jeweils in Prinzipdarstellung.

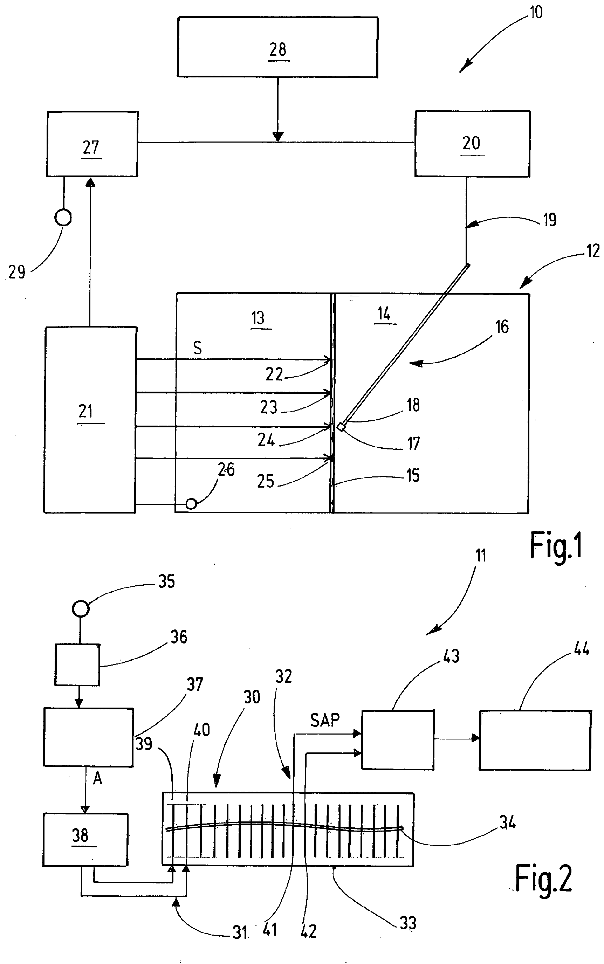

In Figur 1 ist ein Teil einer Einrichtung 10 zur Quantifizierung neuromuskulärer Reizungen außerhalb eines lebenden Organismus veranschaulicht, die nachfolgend als NMR-Teststand bezeichnet wird. Ein zweiter Teil 11 des NMR-Teststands ist in Figur 2 veranschaulicht.

Zu dem NMR-Teststand 10 nach Figur 1 gehört eine Instrumenten-Testkammer 12, die zwei Abteilungen 13, 14 enthält, die voneinander durch eine Wand 15, vorzugsweise eine semipermeable Wand 15 getrennt sind. Die Wand 15 kann insbesondere aus einem explantierten biologischen Material, wie beispielsweise einer Schweinsblase, oder anderem biologischem Gewebe bestehen. Alternativ kann die Wand 15 auch aus einem feinporösem Kunststoffmaterial, einem Filz oder dergleichen gefertigt sein, sofern sichergestellt ist, das die elektrische Spannungsübertragung zwischen den beiden Abteilungen 13, 14 gleich oder ähnlich der Spannungsübertragung einer Schweinsblase oder anderem zu untersuchendem biologischem Material ist.

Beide Abteilungen 13, 14 sind mit einer Flüssigkeit gefüllt. Die Abteilung 13 ist dabei vorzugsweise mit einem Elektrolyt, insbesondere Kochsalzlösung, zum Beispiel physiologische Kochsalzlösung gefüllt. Die Abteilung 14 ist mit einem Fluid gefüllt, das bei einer realen Operation eingesetzt werden soll, beispielsweise ebenfalls physiologische Kochsalzlösung oder auch ein anderweitiges elektrolytisch leitendes oder nicht leitendes Fluid.

In der Abteilung 14 ist ein chirurgisches Instrument 16 angeordnet, das vorzugsweise als bipolares Instrument ausgebildet ist und als solches eine Elektrode 17 sowie eine Neutralelektrode 18 aufweist. Jedoch können auch andere Instrumente zur Anwendung kommen, die lediglich die Elektrode 17, nicht aber die Neutralelektrode 18 umfassen. In einem solchen Fall ist die Neutralelektrode an anderer Stelle in der Instrumententestkammer 12 beispielsweise in der Abteilung 13 oder in der Abteilung 14 angeordnet.

Das Instrument 16 ist über ein Kabel 19, das zum Beispiel zwei mit der Elektrode 17 und der Neutralelektrode 18 verbundene Leiter enthalten kann, mit einem Generator 20 verbunden, der eine elektrische HF-Spannung an das Instrument 16 abgeben kann. Vorzugsweise ist der Generator 20 dabei darauf eingerichtet, verschiedene HF-Spannungsformen abzugeben, wie sie auch beim Einsatz an einem Patienten in Frage kommen und deren physiologische Wirkung hinsichtlich neuromuskulärer Stimulationen miteinander verglichen werden sollen.

Zu dem NMR-Teststand 10 nach Figur 1 gehört außerdem ein Signaldifferenzverstärker 21, der eine Anzahl von Eingängen aufweist, die mit Ableitelektroden 22, 23, 24, 25 verbunden sind, die in der Abteilung 13 an der von dem Instrument 16 abgewandten Seite der Wand 15 angeordnet sind. Die Ableitelektroden 22, 23, 24, 25 sind dabei vorzugsweise entlang eines Pfades, oder in der Nähe eines Pfades, angeordnet, entlang dessen bei einem Säugetier oder Menschen der Obturatornerv verläuft. Außerdem kann der Signaldifferenzverstärker 21 mit einer Referenzelektrode 26 verbunden sein, die in einiger Distanz, vorzugsweise an der von der Wand 15 abgewandten Seite der Abteilung 13, angeordnet ist. Der Signaldifferenzverstärker 21 kann an eine Speichereinrichtung 27, beispielsweise in Gestalt eines Speicheroszilloskops, angeschlossen sein, um von den Elektroden 22 bis 25 abgeleitete elektrische Signale aufzuzeichnen und, falls gewünscht, auch anzuzeigen. Bei einer Aktivierung des Instruments 16, d.h. Bestromung der Elektrode 17 werden die an den Elektroden 22 bis 25 auftretenden Spannungen erfasst und aufgezeichnet.

Zu dem NMR-Teststand nach Figur 10 kann außerdem ein Schalter 28 gehören, der sowohl mit dem Generator 20 als auch mit dem Speicher 27 verbunden ist, um bei Betätigung das Instrument 16 zu aktivieren und zugleich die Aufzeichnung der an den Elektroden 22 bis 25 auftretenden Potentiale auszulösen. Außerdem kann eine nicht weiter veranschaulichte Vorrichtung vorgesehen sein, um das Instrument 16 auf vorgegebene Weise zu positionieren, d.h. an einer bestimmten Position der Abteilung 14 ruhend zu halten. Die Vorrichtung kann in einer weiteren Ausführungsform auch dazu eingerichtet sein, das Instrument entlang eines vorgegebenen Wegs, zum Beispiel auf die Wand 15 zu und von dieser weg oder entlang der Wand 15, zu bewegen. Die Positionierung und Bewegung des Instrumentes 16 kann dann gleichfalls von dem Schalter 28 gesteuert werden. Der Speicher 27 weist einen Ausgang 29 auf, an dem die von den Elektroden 22 bis 25 erhaltenen und über den Signaldifferenzverstärker 21 verstärkten, in dem Speicher 27 abgespeicherten Signale zur Übergabe an weitere Teile des Teststands bereitstehen oder bereitgestellt werden können.

Der insoweit beschriebene Teil des NMR-Teststands 10 nach Figur 1 arbeitet wie folgt:
Zum reproduzierbaren Erfassen elektrischer Spannungen, die beim Einsatz des Instruments 16 an Gewebe in einem menschlichen oder tierischen Körper auftreten können, werden mit dem NMR-Teststand 10 ein oder mehrere Versuche durchgeführt. Dazu wird zunächst ein zu testendes Instrument 16 sowie an den Generator 20 ein gewünschter Mode, d.h., eine ausgewählte HF-Spannungsform ausgewählt. Zusätzlich zu der Spannungsform können die HF-Leistung und/oder die HF-Spannung sowie bedarfsweise andere elektrische Parameter, wie beispielsweise Maximalstrom, Maximalleistung, das Puls-Pause-Verhältnis der HF-Spannung oder ein Bereich für diese festgelegt werden. Außerdem kann eine Position des Instruments 16 festgelegt werden, in der das Instrument 16 während des Versuchs gehalten wird. Alternativ kann ein Weg festgelegt werden, entlang dessen das Instrument 16 während des Versuchs zu bewegen ist.

Zur Durchführung des Versuchs wird der Schalter 28 betätigt, wonach das Instrument 16 bestromt und zum Beispiel entlang der Wand 15 über eine vorbestimmte Distanz bewegt wird. Die dabei an den Elektroden 22, 23, 24, 25 auftretenden Spannungen werden erfasst, von dem Signaldifferenzverstärker 21 verstärkt (oder abgeschwächt) und in dem Speicher 27 abgespeichert. Der Speicher 27 kann dabei darauf eingerichtet sein, eine größere Anzahl von Signalen abzuspeichern, die von einer größeren Anzahl von Aktivierungen, d.h. Betätigungen des Schalters 28 herrühren.

Wenigstens eines der so gewonnenen Signale S wird nun in dem zweiten Teil 11 des NMR-Teststands auf reproduzierbare Weise auf seine physiologische Wirkung untersucht. Dazu weist der NMR-Teststand 11 nach Figur 2 ein Nervmodell 30 auf, das zum Beispiel durch ein elektrisches Netzwerk gebildet sein kann. Das Nervmodell weist jedenfalls einen Eingang 31 und einen Ausgang 32 auf, der, sofern ein über der Steuerschwelle liegendes Anregungssignal an den Eingang 31 gegeben wird, an dem Ausgang 32 ein Summenaktionspotential (SAP) 32 oder mehrere SAPs liefert. Im vorliegenden Fall wird das Nervmodell 30 durch eine Nerventestkammer 33 gebildet, die eine Reihe von vorzugsweise stäbchenförmigen in vorgegebenem Abstand von beispielsweise 5 mm parallel zueinander orientierten Elektroden aufweist, auf die ein präparierter zum Beispiel einem Tier entnommener Nerv 34 so aufgelegt ist, dass er die stäbchenförmigen Elektroden berührt.

Der in Figur 2 veranschaulichte Teil des NMR-Teststands 11 weist einen Eingang 35 auf, der mit dem Ausgang 29 des NMR-Teststands 10 gemäß Figur 1 verbunden sein kann. Die Verbindung kann jede zur Datenübertragung geeignete Verbindung, einschließlich einer Funkverbindung, einer Lichtstrecke, einer Kabelverbindung oder einer Verbindung über Datenträger, wie beispielsweise CD oder Stick, oder über Netzwerk sein. An den Eingang 35 kann sich eine Verarbeitungseinrichtung 36 anschließen, die beispielsweise der Signalfilterung dienen kann. Zum Beispiel können aus den in dem Speicher 27 abgespeicherten Signalen alle Frequenzanteile oberhalb einer Frequenzgrenze von zum Beispiel 100 kHz ausgesiebt und somit beseitigt werden. Alternativ kann dieser Teil der Signalverarbeitung auch von dem Signaldifferenzverstärker 21 und/oder der Speichereinrichtung 27 übernommen werden.

Der Eingang 35 ist direkt oder die Signalverarbeitungseinrichtung 36 mit einem Signalgenerator 37 verbunden, der direkt oder über einen Signalverstärker 38 mit Anregungselektroden 39, 40 der Nerventestkammer 33 verbunden ist. Der darauf liegende Nerv 34 empfängt das an den Stimulationselektroden 39, 40 anliegende Signal.

An zwei andere als Ableitelektroden 41, 42 ausgewählte Elektroden ist ein Verstärker 43 angeschlossen, dessen Ausgangssignal einem Oszilloskop, einer Datenbank oder einem Speicherblock 44 zugeleitet ist.

Es wird darauf hingewiesen, dass einzelne, mehrere oder alle in Figur 2 dargestellten Elemente des NMR-Teststands 11 nach Figur 2 als elektrisch analoge Schaltung, als digitale Schaltung oder als Computermodell bereitgestellt werden können.

Zum Testen der physiologischen Auswirkung wenigstens eines der von dem NMR-Teststand Teil 10 nach Figur 1 ermittelten Signale wird im einfachsten Fall aus den aufgezeichneten Signalen ein weitgehend störungsfreies Signal ausgewählt werden. Zu nah an der Elektrode 17 aufgenommene Signale können gestört sein. Zu weit entfernt aufgenommene Signale können zu schwach sein. Vorzugsweise wird das Signal derjenigen Elektrode 22 - 25 ausgewählt, die etwa 3 mm von dem Schnitt, d.h. von der Elektrode 17 entfernt ist. In dieser Entfernung treten kaum thermische Schädigungen von Gewebe auf, hingegen sind neuromuskuläre Stimulationen noch zu erwarten.

Das ausgewählte Signal wird dem Nervmodell 30 des NMR-Teststands Teil 11 als Anregungssignal zugeleitet. Dazu wird das an dem Eingang 35 empfangene Signal durch die Signalverarbeitungseinrichtung 36 gewandelt, beispielsweise tiefpassgefiltert, gegebenenfalls mittels des Signalverstärkers 38 verstärkt und den Stimulationselektroden 39, 40 zugeleitet. Der präparierte Nerv 34 reagiert auf diese Anregung mit der Generierung von mehr oder weniger SAPs die von den Ableitelektroden 41, 42, gemessen und schlussendlich in dem Speicherblock 44 erfasst werden. Es können damit die physiologischen Wirkungen verschiedener an dem Eingang 35 bereitgesellten Signale reproduzierbar untersucht und verglichen werden.

Das an den Elektroden 39, 40 anliegende Referenz-Anregungssignal wird hinsichtlich seiner Amplitude vorzugsweise so eingestellt, dass das an den Ableitelektroden 41, 42 empfangene, von dem Nerv 34 gelieferte Summenaktionspotential 32 einem Bruchteil des maximalen SAPs entspricht. Dieser Bruchteil SAP₈₀ ist vorzugsweise 80% des maximalen SAPs.

Es können dem Nervmodell 30 auch nacheinander die Signale mehrerer oder aller Elektroden 22 - 25 zugeleitet werden, um auf diese Weise herauszufinden, in welcher Entfernung vom Schnitt neuromuskuläre Stimulationen noch auftreten und in welcher Entfernung noch sicher geschnitten werden kann.

Das an den Ableitelektroden 41, 42 empfangene Signal kann ein gemischtes Signal sein, das sowohl von dem Nerv 34 übertragene SAPs als auch Signalanteile enthält, die durch direkte elektrische Leitung zustande gekommen sind. Es ist deswegen dienlich, den Nerv 34 durch einen funktionsunfähigen Nerv zu ersetzen, der zwischen den Stimulationselektroden 39, 40 sowie den Ableitelektroden 41, 42 eine Quetschstelle enthält, an der die Reizweiterleitung unterbrochen ist. Das von einem solchen funktionsunfähigen Nerv auf die Ableitelektroden 41, 42 übertragene Signal kann von dem korrespondierenden an den Ableitelektroden 41, 42 gemessenen Signal des funktionsfähigen Nerven 34 subtrahiert werden. Damit kann das von dem Nerv 34 fortgeleitete Signal artefaktfrei erfasst und abgespeichert werden, Stimulusartefakte werden vermieden.

Ein weiterer Teil des erfindungsgemäßen Verfahrens betrifft die Auswahl der Ableitelektroden 41, 42 aus der Gruppe der vorhandenen Elektroden. Dazu wird an die Stimulationselektroden 39, 40 ein definiertes Standardsignal beispielsweise ein 100 µs Rechteckpuls einer solchen Spannung angelegt, die mit Sicherheit zur vollständigen Stimulation des Nerven 34, d.h. sämtlicher enthaltener Nervenfasern, führt, so dass dieser das maximale Summenaktionspotential SAP liefert. Das dazu sicher ausreichende Anregungssignal ist beispielsweise ein 7V-Signal. Es werden nun als Ableitelektroden 41, 42 diejenigen Elektroden ausgewählt, die einen vorgegebenen Abstand von z.B. 1,5 cm zu den Stimulationselektroden 39, 40 haben. Sollten die an den Ableitelektroden eintreffenden Summenaktionspotentiale zu gering sein, d.h. eine vorgegebene Grenze (Amplitude) unterschreiten, kann der Abstand verkürzt werden, z.B. auf 1 cm.

Alternativ können als Ableitelektroden 41, 42 auch diejenigen Elektroden 41, 42 ausgewählt werden, an denen mindestens ein Summenaktionspotential von 3 mV gemessen wird. Wird der Test mit dem in Figur 2 am weitesten rechts liegenden Elektrodenpaar begonnen, werden dazu, falls nicht ein Summenaktionsspotential von wenigstens 3 mV erreicht wird, Ableitelektrodenpaare ausgewählt, die weiter links liegen. Im nächsten Schritt werden Standardrechteckimpulse von zum Beispiel 100 ps Dauer an die Stimulationselektroden 39, 40 gegeben, wobei die Reizamplitude bei einem niedrigen Wert von zum Beispiel 100 mV beginnt und schrittweise erhöht oder erniedrigt wird. Ist die Amplitude, die an den Stimulationselektroden 39, 40 nötig ist, um an den Ableitelektroden 41, 42 überhaupt irgendein ein Summenaktionspotential zu registrieren, größer als 500 mV, werden schrittweise noch weiter links liegende Elektroden als Ableitelektroden gewählt. Auf diese werden diejenigen Elektroden als Ableitelektroden 41, 42 gewählt, mit denen eine standardisierte Messung möglich ist.

Erfindungsgemäß wird ein zweiteiliger NMR-Versuchsstand 10, 11 bereitgestellt, mit dem sich Instrumente 16, Generatorspannungen und/oder Operationsmodi hinsichtlich neuromuskulärer Stimulationen systematisch untersuchen lassen. Durch Trennung des NMR-Versuchsstands in einen ersten Teil 10 zur Anwendung des Instrumentes und in einen zweiten Teil 11 zur Untersuchung der physiologischen Wirkung auf Nerven 34, wird eine artefaktfreie Messung möglich. Die räumliche und zeitliche Trennung der HF-Anwendung von der Aufzeichnung der Summenaktionspotentiale werden HF-Störungen, die sonst von dem Instrument ausgehen und die Messung stören, unwirksam gemacht. Außerdem können die in der Instrumententestkammer gewonnenen elektrischen Signale einer Vorverarbeitung, wie zum Beispiel einer Filterung, einer Verstärkung oder einer Abschwächung unterzogen werden. Die aufgezeichneten von dem Instrument ausgehenden elektrischen Signale können beliebig oft einer Untersuchung an dem Nervmodell 30 unterzogen werden. Die Varianz, die mit der Aufzeichnung der Summenaktionspotentiale bei direktem Einsatz des Instruments verbunden ist, kann so minimiert werden. Außerdem vermeidet der gesonderte Test der erhaltenen Signale an dem Nervmodell 30 eine thermische Schädigung des präparierten Nervs 34 und somit eine Verfälschung von Messergebnissen. Zudem wird mit der Erfindung der apparative Aufwand zur Untersuchung von elektrochirurgischen Systemen hinsichtlich neuromuskulärer Reizungen gemindert.

### Bezugszeichen:

- 10: NMR-Teststand - erster Teil
- 11: NMR-Teststand - zweiter Teil
- 12: Instrumenten-Testkammer
- 13, 14: Abteilungen
- 15: Wand
- 16: Instrument
- 17: Elektrode
- 18: Neutralelektrode
- 19: Kabel
- 20: Generator
- 21: Signaldifferenzverstärker
- 22 - 25: Elektroden
- 26: Referenzelektrode
- 27: Speicher
- 28: Schalter
- 29: Ausgang
- 30: Nervmodell
- 31: Eingang (Nerven-Testkammer)
- 32: Ausgang (SAP-Signal)
- 33: Nerven-Testkammer
- 34: Nerv
- 35: Eingang (Nervmodell)
- 36: Signalverarbeitungseinrichtung
- 37: Signalgenerator
- 38: Signalverstärker
- 39, 40: Stimulationselektroden
- 41, 42: Ableitelektroden
- 43: Verstärker
- 44: Speicherblock

## Patentansprüche

1. Verfahren zur Quantifizierung neuromuskulärer Reizungen außerhalb eines lebenden Organismus, wobei bei dem Verfahren:
a) ein Instrument (16) in einer Instrumenten-Testkammer (12) platziert, mit einer HF-Spannung beaufschlagt und auf vorgegebene Weise positioniert wird,
b) in der Instrumenten-Testkammer (12) mittels mindestens einer Elektrode (22) ein elektrisches Signal (S) aufgenommen wird,
c) aus dem aufgenommenen elektrischen Signal (S) ein Anregungssignal (A) ermittelt wird,
d) ein Nervmodell (30) mit dem Anregungssignal stimuliert und von dem Nervmodell (30) abgegebene Summenaktionspotentiale (SAPs) erfasst werdenwobei das Nervmodell in einer Nerven-Testkammer (30) mit mindestens einem Stimulationselektrodenpaar (39, 40) und mindestens einem Ableitelektrodenpaar (41, 42) so platziert wird, dass es sowohl mindestens ein Stimulationselektrodenpaar (39, 40) als auch die mindestens ein Ableitelektrodenpaar (41, 42) berührt,
wobei das Anregungssignal (A) an die Stimulationselektrode (39, 40) angelegt und ein an den Ableitelektroden (41, 42) eintreffendes Signal als Summenaktionspotentiale (SAPs) erfasst werden.

2. Verfahren nach Anspruch 1, **dadurch gekennzeichnet, dass** als Nervmodell (30) ein Abschnitt eines präparierten Nerven (34) genutzt wird.

3. Verfahren nach einem der vorstehenden Ansprüche, **dadurch gekennzeichnet, dass** das aufgenommene elektrische Signal (S) gespeichert wird.

4. Verfahren nach einem der vorstehenden Ansprüche, **dadurch gekennzeichnet, dass** das aufgenommene elektrische Signal (S) zur Ermittlung des Anregungssignals (A) verarbeitet wird.

5. Verfahren nach Anspruch 4, **dadurch gekennzeichnet, dass** aufgenommene elektrische Signal (S) zur Ermittlung des Anregungssignals (A) einer Tiefpassfilterung unterzogen wird.

6. Verfahren nach einem der vorstehenden Ansprüche, **dadurch gekennzeichnet, dass** das Anregungssignal (A) zwischengespeichert wird.

7. Verfahren nach Anspruch 6, **dadurch gekennzeichnet, dass** das Anregungssignal (A) normiert wird, indem es mit einer Referenzreizspannung abgeglichen wird.

8. Verfahren nach Anspruch 7, **dadurch gekennzeichnet, dass** zur Normierung des Anregungssignals ein Referenz-Anregungssignal an die Stimulationselektrode (39, 40) angelegt und zunächst das Summenaktionspotential (SAP) bestimmt und danach das Referenz-Anregungssignal solange erhöht oder vermindert wird, bis das sich einstellende Summenaktionspotential (SAP) innerhalb festgelegter Grenzen liegt.

9. Verfahren nach Anspruch 8, **dadurch gekennzeichnet, dass** das Anregungssignal (A) mit einem Verstärkungsfaktor verstärkt oder abgeschwächt wird, der der Quotient aus der Amplitude eines Anregungssignals, das einen festgelegten Bruchteil (SAP₈₀) des Summenaktionsaktionspotentials (SAP) liefert, und der ursprünglichen Amplitude des Referenz-Anregungssignals ist.

10. Verfahren nach einem der vorstehenden Ansprüche, **dadurch gekennzeichnet, dass** das Anregungssignal (A) an einen funktionsunfähigen, in die Nerven-Testkammer (33) eingelegten Nerven angelegt und zur Bestimmung des Stimulusartefakts (ART) mit dem Anregungssignal (A) beaufschlagt wird.

11. Verfahren nach Anspruch 10, **dadurch gekennzeichnet, dass** das Stimulusartefakt (ART) des funktionsunfähigen Nerven von den Summenaktionspotentialen (SAPs) des funktionsfähigen Nerven (34) subtrahiert wird und dass das so erhaltene Differenzsignal als Grundlage zur Bewertung des Instruments (16) und/oder der HF-Spannung und oder der Positionierung des Instruments (16) hinsichtlich des Potenzials für neuromuskuläre Reizungen herangezogen wird.

12. Einrichtung zur Quantifizierung neuromuskulärer Reizungen außerhalb eines lebenden Organismus gemäß dem Verfahren nach einem der vorstehenden Ansprüche, mit einer Instrumenten-Testkammer (12), die eine erste Abteilung (13) und eine zweite Abteilung (14) aufweist, wobei die Abteilungen (13, 14) voneinander durch eine Wand (15) voneinander getrennt und jeweils wenigstens teilweise mit einer Flüssigkeit, insbesondere einem Elektrolyt, gefüllt sind, wobei in der ersten Abteilung (14) Ableitelektroden (22, 23, 24, 25) positioniert sind und wobei das zu testende Instrument (16) in der zweiten Abteilung (14) positioniert ist, mit einer Verarbeitungseinheit (36), der von den Ableitelektroden (22, 23, 24, 25) abgegebene Signale (S) zugeleitet sind,
mit einem Signalgenerator (37), der an die Verarbeitungseinheit (36) angeschlossen ist, und
mit einer Nerven-Testkammer (33) zur Aufnahme eines präparierten Nerven (34), die an den Signalgenerator (37) angeschlossen ist.

13. Einrichtung nach Anspruch 12, **dadurch gekennzeichnet, dass** die Wand (15) eine stromleitende Membrane, insbesondere aus präpariertem biologischem Material ist.

14. Einrichtung nach Anspruch 12, **dadurch gekennzeichnet, dass** die Nerven-Testkammer (33) zwei Stimulationselektroden (39, 40) und mindestens zwei Ableitelektroden (41, 42) aufweist, die vorzugsweise als gerade, in einer gemeinsamen Ebene parallel zueinander angeordnete Stäbe ausgebildet sind.

15. Einrichtung nach Anspruch 12, **dadurch gekennzeichnet, dass** der funktionsunfähige Nerv zur Unterbrechung der Reizweiterleitung eine Quetschstelle aufweist.

## Claims

1. Method for quantifying neuromuscular stimulations external from a living organism comprising the following steps:
a) An instrument (16) located in an instrument test chamber (12) is applied with a radio frequency voltage and is positioned in a defined manner,
b) An electrical signal (S) is recorded in the instrument test chamber (12) by means of at least one electrode (22),
c) A stimulation signal (A) is determined from the recorded electrical signal (S),
d) A nerve model (30) is stimulated with a stimulation signal and compound action potentials (CAPs) output from the nerve model (30) are detected, wherein the nerve model (30) is placed in a nerve test chamber (33) comprising at least one stimulation electrode pair (39, 40) and at least one diverting electrode pair (41, 42), such that it contacts the at least one stimulation electrode pair (39, 40) as well as the at least one diverting electrode pair (41, 42), wherein the stimulation signal (A) is applied to the stimulation electrode (39, 40) and a signal arriving at the diverting electrodes (41, 42) is detected as compound action potentials (CAPs).

2. Method according to claim 1, **characterized in that** a section of a prepared nerve (34) is used as nerve model (30).

3. Method according to any of the preceding claims, **characterized in that** the recorded electrical signal (S) is stored.

4. Method according to any of the preceding claims, **characterized in that** the recorded electrical signal (S) is processed for determination of the stimulation signal (A).

5. Method according to claim 4, **characterized in that** the recorded electrical signal (S) is subject to a low pass filtering for determination of the stimulation signal (A).

6. Method according to any of the preceding claims, **characterized in that** the stimulation signal (A) is buffered.

7. Method according to claim 6, **characterized in that** the stimulation signal (A) is standardized by referencing it with a reference stimulation voltage.

8. Method according to claim 7, **characterized in that** for standardizing the stimulation signal a reference stimulation signal is applied to the stimulation electrode (39, 40) and first the compound action potential (CAP) is determined and subsequently the reference stimulation signal is increased or decreased until the resulting compound action potential (CAP) is within predetermined limits.

9. Method according to claim 8, **characterized in that** the stimulation signal (A) is amplified or attenuated with an amplification factor being the quotient of the amplitude of a stimulation signal providing a predefined fraction (CAP₈₀) of the compound action potential (CAP) and the initial amplitude of the reference stimulation signal.

10. Method according to any of the preceding claims, **characterized in that** the stimulation signal (A) is applied to a non-functional nerve inserted in the nerve test chamber (33), wherein the nerve is applied with the stimulation signal for determination of a stimulus artifact (ART).

11. Method according to claim 10, **characterized in that** the stimulus artifact (ART) of the non-functional nerve is subtracted from the compound action potentials (CAPs) of the functional nerve and that the difference signal obtained in this manner is used as basis for evaluation of the instrument (16) and/or the radio frequency voltage and/or the positioning of instrument (16) in view of the potential for neuromuscular stimulations.

12. Device for quantification of neuromuscular stimulations external from a living organism according to the method according to one of the preceding claims, having an instrument test chamber (12) that comprises a first compartment (13) and a second compartment (14), wherein the compartments (13, 14) are separated from each other by a wall (15), each being at least partly filled with liquid, particularly an electrolyte, wherein in the first compartment (13) diverting electrodes (22, 23, 24, 25) are positioned and wherein the instrument (16) to be tested is positioned in the second compartment (14), having a pre-processing unit (36) to which signals (S) are supplied output from the diverting electrodes (22, 23, 24, 25), having a signal generator (37) that is connected to the pre-processing unit (36) and having a nerve test chamber (33) for reception of a prepared nerve (34), wherein a signal generator (37) is connected to the nerve test chamber (33).

13. Device according to claim 12, **characterized in that** the wall (15) is a current conducting membrane, particularly made of prepared biological material.

14. Device according to claim 12, **characterized in that** the nerve test chamber (33) comprises two stimulation electrodes (39, 40) and at least two diverting electrodes (41, 42) that are preferably configured as sticks arranged in a common plane parallel to each other.

15. Device according to claim 12, **characterized in that** the non-functional nerve comprises a bruise location for interruption of stimulation conduction.

## Revendications

1. Procédé de quantification de stimulations neuromusculaires à l'extérieur d'un organisme vivant, sachant que selon le procédé :
a) un instrument (16) est placé dans une chambre d'essai d'instrument (12), est soumis à une tension HF et est positionné d'une manière prédéfinie,
b) un signal électrique (S) est recueilli dans la chambre d'essai d'instrument (12), à l'aide d'au moins une électrode (22),
c) un signal d'excitation (A) est déterminé à partir du signal électrique (S) recueilli,
d) un modèle de nerf (30) est stimulé avec le signal d'excitation (A), et les potentiels d'action globaux (SAPs) émis par le modèle de nerf (30) sont recueillis, le modèle de nerf étant placé dans une chambre d'essai de nerf (30), comportant au moins une paire d'électrodes de stimulation (39, 40) et au moins une paire d'électrodes de dérivation (41, 42), de manière à ce qu'il soit en contact aussi bien avec au moins une paire d'électrodes de stimulation (39, 40) qu'avec la paire d'électrodes de dérivation (41, 42), au nombre d'au moins une,
le signal d'excitation (A) étant appliqué à l'électrode de stimulation (39, 40), et un signal arrivant aux électrodes de dérivation (41, 42) étant recueilli en tant que potentiels d'action globaux (SAPs).

2. Procédé selon la revendication 1, **caractérisé en ce que** l'on utilise comme modèle de nerf (30) un segment d'un nerf préparé (34).

3. Procédé selon une des revendications précédentes, **caractérisé en ce que** le signal électrique (S) recueilli est enregistré.

4. Procédé selon une des revendications précédentes, **caractérisé en ce que** le signal électrique (S) recueilli est traité en vue de déterminer le signal d'excitation (A).

5. Procédé selon la revendication 4, **caractérisé en ce que** le signal électrique (S) recueilli est soumis à un filtrage passe-bas en vue de déterminer le signal d'excitation (A).

6. Procédé selon une des revendications précédentes, **caractérisé en ce que** le signal d'excitation (A) fait l'objet d'un enregistrement intermédiaire.

7. Procédé selon la revendication 6, **caractérisé en ce que** le signal d'excitation (A) est normalisé en le comparant à une tension de stimulation de référence.

8. Procédé selon la revendication 7, **caractérisé en ce que** pour la normalisation du signal d'excitation, un signal d'excitation de référence est appliqué à l'électrode de stimulation (39, 40) et on détermine d'abord le potentiel d'action global (SAP), puis on augmente ou on réduit le signal d'excitation de référence jusqu'à ce que le potentiel d'action global (SAP) obtenu se situe dans des limites définies.

9. Procédé selon la revendication 8, **caractérisé en ce que** le signal d'excitation (A) est amplifié ou atténué avec un facteur de gain qui est le quotient de l'amplitude d'un signal d'excitation, qui fournit une fraction (SAP₈₀) définie du potentiel d'action global (SAP), et de l'amplitude initiale du signal d'excitation de référence.

10. Procédé selon une des revendications précédentes, **caractérisé en ce que** le signal d'excitation (A) est appliqué à un nerf dysfonctionnel placé dans la chambre d'essai de nerf (33), et le signal d'excitation (A) est appliqué aux fins de déterminer l'artefact de stimulation (ART).

11. Procédé selon la revendication 10, **caractérisé en ce que** l'artefact de stimulation (ART) du nerf dysfonctionnel est soustrait des potentiels d'action globaux (SAPs) du nerf fonctionnel (34), et **en ce que** le signal différentiel ainsi obtenu est utilisé pour l'évaluation de l'instrument (16) et/ou de la tension HF et/ou du positionnement de l'instrument (16) par rapport au potentiel pour des stimulations neuromusculaires.

12. Dispositif de quantification de stimulations neuromusculaires à l'extérieur d'un organisme vivant, conformément au procédé selon une des revendications précédentes,
comprenant une chambre d'essai d'instrument (12) qui présente un premier compartiment (13) et un deuxième compartiment (14), les compartiments (13, 14) étant séparés l'un de l'autre par une paroi (15) et étant chacun remplis au moins partiellement avec un liquide, notamment un électrolyte, des électrodes de dérivation (22, 23, 24, 25) étant positionnées dans le premier compartiment (14), et l'instrument (16) à tester étant positionné dans le deuxième compartiment (14),
comprenant une unité de traitement (36) à laquelle sont transmis des signaux (S) délivrés par les électrodes de dérivation (22, 23, 24, 25),
comprenant un générateur de signal (37) qui est raccordé à l'unité de traitement (36), et
comprenant une chambre d'essai de nerf (33) qui est destinée à accueillir un nerf préparé (34) et qui est raccordée au générateur de signal (37).

13. Dispositif selon la revendication 12, **caractérisé en ce que** la paroi (15) est une membrane conductrice de l'électricité, constituée notamment d'un matériau biologique préparé.

14. Dispositif selon la revendication 12, **caractérisé en ce que** la chambre d'essai de nerf (33) présente deux électrodes de stimulation (39, 40) et au moins deux électrodes de dérivation (41, 42) qui sont réalisées de préférence sous forme de tiges rectilignes, disposées parallèlement les unes aux autres dans un plan commun.

15. Dispositif selon la revendication 12, **caractérisé en ce que** le nerf dysfonctionnel présente un point de compression en vue de l'interruption de la transmission des stimulations.
